# FASCICULE DE BREVET EUROPEEN

(11) **EP 3 393 984 B1**
(45) Date de publication et mention de la délivrance du brevet: **28.04.2021**
(21) Numéro de dépôt: 16826129.5
(22) Date de dépôt: 21.12.2016
(51) Int. Cl.: C02F 11/04, C02F 3/28, C12M 1/107

(54) **INSTALLATION ET PROCÉDÉ DE TRAITEMENT BIOLOGIQUE DE DÉCHETS ET EFFLUENTS ORGANIQUES**
ANLAGE UND VERFAHREN ZUR BIOLOGISCHEN BEHANDLUNG VON ORGANISCHEN ABFÄLLEN UND ABWASSER
FACILITY AND METHOD FOR BIOLOGICALLY TREATING ORGANIC WASTE AND EFFLUENTS

(30) Priorité: 21.12.2015 FR 1562898
(43) Date de publication de la demande: 31.10.2018
(73) Titulaire: Jua Group, Moka (MU)
(72) Inventeur: SALLUSTRO, Jean-Luc, Trou-aux-Biches (MU)
(74) Mandataire: Schmidt, Martin Peter
(86) Numéro de dépôt international: PCT/FR2016/053592
(87) Numéro de publication internationale: WO 2017/109398

(56) Documents cités:
- WO-A2-2011/017420
- US-A- 4 780 415
- US-A- 5 560 819

## Description

### Domaine technique de l'invention

La présente invention concerne une installation de traitement par biodigestion de déchets et effluents organiques, ainsi qu'un procédé de mise en œuvre de cette installation. L'invention est relative à l'optimisation de protocoles de biométhanisation de substrats, déchets et effluents organiques dans un digesteur multiphases à alimentation semi-continue de type piston, acceptant une haute teneur en matière organiques volatile sous conditions thermophiles. L'invention porte en particulier sur la géométrie des différents éléments constitutifs de cette installation, ainsi que sur la dynamique des échanges entre ces éléments constitutifs.

### Etat de la technique

Le traitement de matières organiques par biodigestion est avant tout soumis à des contraintes biologiques que tentent de respecter les techniques qui visent à créer et maintenir un écosystème favorable pour les micro-organismes particuliers à ce type de bio-oxydation. En simplifiant on peut dire d'un biodigesteur qu'il accueille et entretient dans un système de bioréacteurs des populations de microbes strictement anaérobies qui sont amenées à croître et à se reproduire sur un substrat organique constitué de déchets ou plus généralement de matières liquides ou solides mises en solution. Pour l'essentiel ces populations microbiennes particulières développent une activité de bio-oxydation, mais en l'absence de l'oxygène de l'air. La réaction n'est possible que lorsque les trois communautés bactériennes typiques de ce trophisme constituent un écosystème équilibré de telle sorte que l'essentiel des équivalents réducteurs (atomes de carbone et d'hydrogène) produits comme déchets au cours de l'anabolisme bactérien (hydrolyse puis acidophile et acétogénèse) se retrouvent finalement dans le méthane (CH₄, méthanogénèse). Les espèces bactériennes concernées sont complexes et relativement variées mais on connaît assez bien leurs caractéristiques biochimiques et les grands traits de leur écologie. Elles se classifient généralement en trois groupes:
- Les bactéries hydrolytiques et fermentatives.
- Les bactéries acétogènes.
- Les bactéries méthanogènes.

La gestion de l'écosystème artificiel que constitue un bioréacteur anaérobie nécessite donc que l'on intervienne dynamiquement pour assurer certaines conditions physico-chimiques essentielles, telles que le pH, la température et le potentiel d'oxydoréduction, ainsi que les besoins nutritionnels.
- Le pH: Le pH optimum de la digestion anaérobie se situe autour de la neutralité. Il est le résultat du pH optimum de chaque population bactérienne: celui des bactéries acidifiantes se situe entre 5,5 et 6, les acétogènes préfèrent un pH proche de la neutralité tandis que les méthanogènes ont une activité maximale dans une gamme de pH comprise entre 6 et 8. Toutefois, la méthanisation peut se produire dans des milieux légèrement acides ou alcalins.
- La température: L'activité du consortium méthanogène est étroitement liée à la température. Deux plages de températures optimales peuvent être définies: la zone mésophile (autour de 35°C) et la zone thermophile (entre 55-60°C) avec une décroissance de l'activité de part et d'autre de ces températures. La majorité des espèces bactériennes a été isolée dans des environnements mésophiles, mais tous les groupes trophiques des étapes de digestion anaérobie possèdent des espèces thermophiles utilisant les mêmes voies métaboliques que les bactéries mésophiles avec des performances analogues ou supérieures. Il reste néanmoins possible de travailler à des températures différentes des optima avec des performances plus faibles ou plus élevées.
- Le potentiel d'oxydoréduction : Ce paramètre représente l'état de réduction du système, il affecte l'activité des bactéries méthanogènes. Ces bactéries exigent en effet, outre l'absence d'oxygène, un potentiel d'oxydoréduction inférieur à 330 mV pour initier leur croissance.
- Les besoins nutritionnels et métaboliques : Comme tout micro-organisme, chaque bactérie constituant la flore méthanogène demande un apport suffisant de macro-éléments (C, N, P, S) et d'oligo-éléments pour sa croissance. Les besoins en macro-éléments peuvent être évalués grossièrement à partir de la formule brute décrivant la composition d'une cellule (C₅H₉O₃N). Pour les bactéries méthanogènes, le milieu de culture doit avoir des teneurs en carbone (exprimée en Demande Chimique en Oxygène ou DCO), en azote et en phosphore au minimum dans les proportions DCO/N/P égale à 400/7/1.
   L'ammonium est leur principale source d'azote. Certaines espèces fixent l'azote moléculaire alors que d'autres ont besoin d'acides aminés. Les besoins en azote représentant 11 % de la masse sèche volatile de la biomasse et les besoins en phosphore 1/5 de ceux de l'azote.
   Les bactéries méthanogènes possèdent de hautes teneurs en protéines Fe-S qui jouent un rôle important dans le système transporteur d'électrons et dans la synthèse de coenzymes. Aussi la concentration optimale de soufre varie-t-elle de 1 à 2 mM (mmol/L) dans la cellule. Cette flore utilise généralement les formes réduites comme le sulfure d'hydrogène. Les méthanogènes assimilent le phosphore sous forme minérale.
   Certains oligo-éléments sont nécessaires à la croissance des méthanogènes. Il s'agit plus particulièrement du nickel, du fer et du cobalt. En effet, ce sont des constituants de coenzymes et de protéines impliquées dans leur métabolisme. Le magnésium est essentiel puisqu'il entre en jeu dans la réaction terminale de synthèse du méthane ainsi que le sodium apparaissant dans le processus chimio-osmotique de synthèse de l'Adénosine Tri-Phosphate (ATP).
   Il existe des facteurs de croissance stimulant l'activité de certains méthanogènes: acides gras, vitamines ainsi que des mélanges complexes comme l'extrait de levure ou la trypticase peptone.En conclusion, si l'on maîtrise aujourd'hui correctement le « macro-modèle » qui simule un processus de biodigestion, au point que l'on peut prédire sommairement l'ampleur et la forme des productions méthaniques et de la composition d'un digestat ou méthacompost, il n'en reste pas moins que les procédés sont difficiles à mettre en œuvre. En effet, si l'on veut traiter un effluent organique donné, la fraction fermentescible des déchets ménagers et assimilés ou certains déchets organiques industriels ou issus des secteurs agricoles, ou encore un mélange d'intrants (co-digestion) il faut à chaque fois dédier le processus pour atteindre la meilleure productivité. En effet, à chaque substrat correspond un écosystème microbiologique optimum et les créneaux de rendement biochimique sont étroits. En d'autre termes, le défi consiste à concevoir et à mettre en œuvre un digesteur méthanique à faible investissement et faibles coûts d'exploitation mais parfaitement versatile, pour assurer une productivité méthanique élevée quelle que soit la variation des contraintes d'intrants.

Or l'approche empirique et analytique récente dont bénéficie la biodigestion, plutôt que la versatilité, a permis de dégager un certain nombre de lois fonctionnelles qui sont appliquées à des technologies de biodigestion variées mais non pas variables, chacune ayant une adaptation privilégiée au traitement d'un certain type de déchets, en quantités variables avec des qualités de méthacompost et de biogaz différentes.

### Les différents procédés

Un bioréacteur sous condition anaérobie est un artefact qui tente d'optimiser les conditions de vie d'une colonie de micro-organismes donnée à un moment donné et/ou en un lieu donné afin de concentrer en un minimum de temps de rétention biologique, donc dans un volume de bioréacteur minimal, la production maximale de méthane qui résulte de la digestion des substrats mis en solution aqueuse ou plus généralement en absence d'oxygène gazeux. En simplifiant, disons qu'un biodigesteur est constitué de quatre composants majeurs:
- Une enceinte étanche et souvent calorifugée
- Un dispositif d'agitation ou de brassage
- Un dispositif de chauffage des digestats
- Des dispositifs d'entrée et de sortie pour le substrat, le digestat et le biogaz.

Selon les procédés mis en œuvre on distingue deux grands types d'écosystèmes:
- Biomasse fixée
- Biomasse libre

Dans un digesteur à biomasse fixée, l'enceinte sert non seulement à contenir le substrat et l'isoler de l'air, mais aussi à fixer des colonies bactériennes anaérobies sur des supports adaptés. Certaines techniques en phase liquide utilisent des cellules fixatrices autonomes qui sont immergées dans le flux. D'une manière générale, l'avantage de ce procédé réside dans le maintien de la disponibilité des souches bactériennes malgré le transfert permanent ou séquentiel des flux de substrats traités, l'objectif recherché étant de ne pas avoir à redémarrer un ensemencement bactérien ou d'éviter de spécialiser la flore par apports chimiques. Plusieurs types de procédés fixants sont disponibles, certains par exemple granulent le substrat ou une partie du substrat entrant avant de l'ensemencer et le faire circuler dans l'enceinte du biodigesteur.

En règle générale, les opérations de bio-oxydation des déchets ou matières organiques doivent satisfaire plusieurs critères d'efficacité et de biosécurité que l'on opère en réalisant des réglages et des ajustements critiques. Ainsi, dans un digesteur à biomasse libre ou fixée, on fait appel à des procédés de renforcement de la biomasse active qui résultent du réchauffage et de la circulation des jus et, éventuellement, d'apports en oligo-éléments et en correcteurs de pH. Le procédé est adaptatif et fait le pari de la capacité spontanée de la flore bactérienne à se spécialiser en fonction des contraintes du milieu, notamment en ce qui concerne la présence de nutriments en quantité importante. L'adaptabilité de la biomasse, laissée libre de quitter l'enceinte avec l'écoulement séquentiel ou continu des flux, et d'évoluer en fonction des contraintes de l'écosystème, est renforcée par des actions « extérieures », thermiques (maintien en conditions mésophiles à 36°C ou thermophiles à 55°C), chimiques (neutralisation des pH acides ou alcalins) et mécaniques (transferts, fluidification et brassage). En règle générale un biodigesteur nécessite donc soit un bon suivi des indications fournies par des capteurs, afin de permettre une réponse humaine de réglage en temps différé, soit l'analyse et le traitement automatique des signaux transmis par des capteurs inférant en temps réel l'actionnement d'effecteurs.

Au-delà de la différenciation entre biomasse fixée et population libre, réglages manuels ou automatisés, on distingue aussi deux types de dynamique des flux:
- Chargement séquentiel.
- Alimentation continue.

Les procédés à chargement séquentiel ont comme caractéristique majeure qu'ils cherchent à établir, dans la même enceinte pour une seule dose de substrat, la succession des phases majeures de la digestion méthanique. Pour le dire autrement on peut considérer que, dans ce contexte, les populations bactériennes évoluent sur un substrat identique du début à la fin du cycle et n'ont donc pas besoin de dépenser de l'énergie pour s'adapter à des changements non attendus de leur écosystème, ce sont elles qui le transforment et non pas l'inverse. Ainsi dès lors que le chargement de la cuve est achevé, et il peut se faire en une journée comme en trois ou quatre, les conditions optimum de démarrage de la phase d'hydrolyse sont apportées (température, pH, nutriments, ensemencement). C'est ensuite le tour de la phase transitoire d'acidogénèse qui est régulée pour permettre le déclenchement de l'acétogénèse et enfin de la méthanogénèse. En théorie ce procédé présente l'intérêt d'avoir un Temps de Rétention Hydraulique (TRH) plus court que celui des protocoles en flux continu et d'être plus facile à maîtriser. D'une manière générale il faut disposer de plusieurs cuves fonctionnant en parallèle qui sont activées l'une après l'autre au fur et à mesure de leur remplissage. En cas de dysfonctionnement d'une cellule on peut continuer le traitement avec les autres. C'est aussi un procédé où les cuves sont plus petites et qui acceptent généralement des substrats plus denses en matière sèche. Néanmoins, le chargement séquentiel oblige à multiplier les enceintes et les dispositifs annexes tels que les trémies de chargement, les valves et autres pompes.

L'alimentation continue s'oppose strictement au chargement séquentiel sur plusieurs plans. D'abord parce que l'écosystème et particulièrement la flore bactérienne sont amenés à être polyvalents, ou plutôt à faire coexister dans la même enceinte et en même temps, mais pas forcément dans la même couche ou étage du volume de bioréaction, des bactéries et leurs co-enzymes pour les quatre phases du cycle. Ensuite parce que, pour obtenir un TRH suffisant, il faut dimensionner la cuve sur des volumes très importants. Cela entraîne des dépenses énergétiques proportionnelles pour maintenir une température adaptée (très rarement thermophile sauf sur de petites unités) et surtout pour brasser le mélange en continu, afin d'éviter la formation d'une croûte en surface et de sédiments trop denses au fond de la cuve. Il faut néanmoins noter que ce procédé, très ancien puisque les biodigesteurs domestiques ou fermiers chinois sont majoritairement alimentés en continu, s'adapte bien aux micro-gisements de substrats organiques homogènes à très faible variabilité. En effet avec des dimensions très faibles (quelques dizaines de m³), des déchets de qualité et quantité stable, ils sont faciles à entretenir si tant est qu'on ne cherche pas à évacuer les sédiments en temps réel mais plutôt les flux en phase liquide ou turbide (appelés éluats) qui peuvent être ensuite valorisés en épandage. Néanmoins après plusieurs cycles de fonctionnement ces petites unités doivent être arrêtées et vidangées de leurs sédiments qui, à force de s'accumuler, réduisent le volume utile de l'installation et nuisent au développement de la flore bactérienne. Seuls certains procédés industriels arrivent à produire en plus du biogaz, des flux très chargés dont on extrait par décantation et/ou essorage des méthacomposts (digestats) valorisables en tant que fertilisant biologique solide. L'avantage de ce procédé, au niveau industriel ou domestique, réside donc essentiellement dans sa capacité à accepter un flux continu de déchets ou d'effluents à faible charge organique avec des productions de biogaz moyennes mais une valorisation possible des effluents extraits et plus difficilement du méthacompost.

Sur la base de ce que nous venons de décrire, deux types majeurs de procédés continuent de se concurrencer :
- Phase unique.
- Phases différenciées.

Dans le premier cas, que le biodigesteur soit de type séquentiel ou continu, à biomasse fixée ou libre, l'ensemble des phases se déroule dans la même enceinte. Ce sous-système est soit gravitaire (sédimentation) soit à contre flux et il est largement majoritaire. Les variations technologiques fondamentales concernent les modalités du mélange séquentiel ou linéaire des substrats (brassé, par opposition à pulsé et infiniment mélangé), les modalités d'introduction des substrats et d'extraction des digestats et éluats.

Dans le second cas et en théorie, chacune des quatre phases peut être confinée dans une cuve distincte et le passage du substrat modifié à l'issue de chaque phase vers la suivante est assuré par un système mécanique ou hydraulique. En réalité l'état de l'art favorise nettement les systèmes bi-phase au sein desquels hydrolyse et acidogénèse sont confinées dans une première enceinte alors que l'acétogénèse et la méthanogénèse sont assurés ensemble dans la seconde enceinte. Le but recherché par ces procédés multiphases est de mieux gérer les phases individuellement en jouant sur les micro-conditions optimisant ces différents écosystèmes. Plus complexes et coûteux, les procédés à phases différenciées ont néanmoins un meilleur rendement en termes de biodégradabilité notamment pour des substrats qui requièrent une forte spéciation enzymatique et / ou un environnement chimique ou thermique particulier. Par contre pour un flux de déchets homogènes dans le temps et d'une composition n'offrant pas de risques particuliers (surtout au stade de l'acétogénèse) il est généralement considéré que ce procédé n'apporte pas de valeur ajoutée suffisante pour légitimer la complexité et l'investissement requis.

Enfin, on fait la distinction entre trois types de biodigesteurs selon la concentration de Solides Totaux mis en Suspension (STS) dans les flux, c'est à dire la proportion de matière sèche (MS) mise en solution dans le digesteur:
- Faible concentration de MS avec moins de 10 % de STS.
- Concentration moyenne de MS contenant entre 15% et 20%, de STS.
- Haute concentration en MS contenant entre 22% et 40% de STS.

Les applications du principe de la biodigestion de flux à faible teneur en STS ont comme intrants principaux des effluents industriels ou domestiques, comme c'est le cas par exemple pour les stations de traitement des eaux usées urbaines. Les biodigesteurs qui s'appliquent à traiter ce flux ont une configuration particulière, le principe consiste à utiliser le biodigesteur comme une cuve de sédimentation où les STS sont retenus et traités par voie anaérobie alors qu'un flux d'eau épuré s'en échappe. Plus clairement dit, le Temps de Rétention Biologique (TRB) des STS y est plus important que celui du flux entrant en termes de Temps de Rétention Hydraulique (TRH) car le biodigesteur intègre un système de décantation passive ou active et un système de rétention/dégradation anaérobie des MS digestibles.

En tant que tels ces biodigesteurs sont impropres au traitement des déchets organiques solides sauf à ce que ces derniers soient broyés et mis en solution avec des effluents qui constitueront toujours la majorité de l'intrant. Sous ces protocoles, la production de biogaz et de méthacompost (en l'occurrence sous forme de boues) est relativement faible mais leur capacité d'épuration primaire d'un effluent est très bonne et leur bilan énergétique s'équilibre avec la cogénération du biogaz. Éventuellement la productivité de ces sous-systèmes s'améliore avec la revente des liqueurs de digestats (éluats) en tant que fertilisants organiques liquides. Les charges volumiques maximales applicables sont de l'ordre de 2 à 5 kg de DCO/ m³/j.

Le type de biodigesteur à concentration moyenne en STS est le plus courant, sous cette configuration un substrat digestible solide est mis en solution dans 2 fois à 3 fois son poids en eau. Cette modalité de densité en matière organique mise en solution correspond à une recherche d'équilibre entre la quantité de matière digestible, sa viscosité et sa coalescence dans l'enceinte du digesteur et la capacité du milieu anaérobie à abriter et maintenir des populations bactériennes dégradatrices sans risquer leur inhibition par voie de saturation biochimique. En effet, pour que l'activité bactérienne opère dans les meilleures conditions il est nécessaire que le digestat ne se compacte pas tant qu'il peut être mobilisé au fur et à mesure des différentes phases de la biodigestion. Ce procédé s'adapte donc au traitement de la fraction digestible des déchets organiques solides sous réserve d'un tri efficace en amont pour évacuer les indésirables et d'un broyage relativement fin qui autorise le transfert hydraulique de la masse digestible et la prolifération d'une forte diversité bactérienne. Plus approprié aux procédés à chargement continu que séquentiel le principe de la concentration moyenne en MS profite particulièrement des systèmes à biomasse fixée car le flux de substrat a un débit suffisamment important pour appauvrir les flores résidentes. D'une manière générale, les charges volumiques à appliquer peuvent atteindre 15 à 20 kg DCO/m³/j Les temps de séjour hydraulique varient entre 4 et 5 semaines. Sous cette configuration les rendements en biogaz sont bons et la production de méthacompost sous forme de matière fibrée sédimentée est correcte mais nécessite pour le moins une décantation si ce n'est une centrifugation.

Certains gisements de déchets organiques sont constitués d'une fraction solide importante avec une digestibilité faible. En clair la masse de MS est importante mais la proportion de Matière Organique Volatile (MOV) sur la MS est peu importante. Dans la mesure où l'on ne peut valablement concentrer la MOV de ces déchets il est opportun de disposer d'une technologie qui autorise leur traitement par voie anaérobie et certains biodigesteurs sont conçus pour ce type d'application. Ils sont dits à Haute concentration en MS.

La spécificité de ces applications réside dans le mode d'avancement et de brassage du substrat et dans le fait qu'il s'agit presque exclusivement de bioréacteurs à chargement séquentiel et à biomasse libre, mais avec ensemencement. D'une manière générale, il faut noter qu'au-delà d'un certain seuil de teneur en MOV, il existe un risque de surcharge qui peut entraîner une inhibition de la méthanogénèse ce qui est surtout valable pour les déchets riches en protéines animales (carcasses et graisses). De plus les charges volumiques à appliquer peuvent atteindre 40 kg DCO/m³/j. Les temps de séjour hydraulique varient entre 2 et 3 semaines.

Il faut ainsi prendre en compte le fait qu'au-delà de 3 g/L, l'ammonium (NH₄⁺) est un inhibiteur de la méthanogénèse. On sait aussi que cette limite de 3 g/L de NH₄⁺ ne doit pas être dépassée pour des déchets dont le rapport C / N est égal ou inférieur à 20 avec un taux de MOV de l'ordre de 60% de la MO.

La technique la plus pratiquée pour maintenir les substrats organiques en deçà de ce seuil consiste à mélanger les déchets trop riches en protéines (viscères, poisson, produits laitiers, carcasses et autres déchets carnés) avec des substrats carbonés. L'alternative à l'approche par régulation du mélange consiste à abattre le taux de MOV des déchets (surtout la proportion d'ammonium) en les soumettant à une phase préalable de fermentation aérobie thermophile intense, mais celle-ci requiert de toute façon que les déchets carnés soient mélangés à des substrats carbonés.

On connaît, par WO-A-2011/017420, un bioréacteur formé de deux modules placés en série, dont chacun comprend des compartiments placés côte à côte. La matière organique à traiter est admise, via un tube d'entrée, en partie basse du premier module. Elle circule ensuite le long des compartiments de ce module, sous l'effet présumé d'un courant gazeux injecté en partie basse de chacun de ces compartiments et qui est réputé créer une convection ascendante. Lors de ce trajet dans le premier module, de l'acétate est formé. Le substrat est ensuite admis dans le deuxième module, où il suit un cheminement analogue le long de ses compartiments constitutifs. En aval de ce deuxième module, il y a création de méthane.

Cette solution bi-phasique connue présente cependant certains inconvénients. En effet, l'installation décrite dans WO-A-2011/017420 n'est pas satisfaisante pour le traitement de substrats à haute teneur en solides, mais convient plutôt à des substrats fortement dilués. En effet, le mode de brassage obtenu du fait du passage d'une alvéole à l'autre ne pourrait convenir à des matériaux fortement turbides dont la sédimentation serait accrue, du fait des turbulences créées au bas du système et sur les bords d'écoulement en partie haute. Cette installation est sensible aux phénomènes de colmatage, dès que la turbidité atteint un seuil critique. Seule une fraction liquide emportant avec elle des fines légères d'une portance suffisante pour être mues par le courant gazeux peut traverser intégralement l'installation, alors que la partie plus lourde a tendance à sédimenter en partie basse, ce qui crée des zones de blocage qui s'opposent rapidement au bon écoulement du courant gazeux. Cette installation nécessite la mise en place d'un courant gazeux puissant et rapide, ce qui a tendance à générer des turbulences fortes et, par conséquent, à nuire à la mise en place des réactions biochimiques appropriées. De plus un tel courant à forte vélocité n'autorise pas un temps de rétention biologique (TRB) suffisant.

On connaît en outre, du brevet américain 4,780,415, un procédé et une installation permettant de réaliser une dégradation en milieu anaérobie de produits organiques. Cette installation comprend un réacteur, qui est divisé en deux zones réactionnelles par une paroi verticale. La zone réactionnelle amont est alimentée par une région tubulaire, dans laquelle les substrats alimentés s'écoulent de haut en bas. La circulation des substrats, entre les zones réactionnelles, suit une forme de dôme. Enfin, les substrats sont évacués dans une région tubulaire aval, en s'écoulant de bas en haut. Cette circulation le long du réacteur est assurée de manière pneumatique. On notera cependant que ce document enseigne d'utiliser un réacteur unique et que, de plus, les zones réactionnelles sont disposées les unes à côté des autres.

### Objets de l'invention

Compte tenu de ce qui précède, l'invention vise à remédier aux inconvénients de l'art antérieur évoqués ci-dessus. Elle vise en particulier à proposer une installation dont la compacité est améliorée, qui est plus robuste notamment car elle se compose d'un nombre réduit d'éléments constitutifs, et dont le rendement est amélioré. L'invention vise également à proposer une telle installation, qui privilégie radicalement la versatilité et la modularité des moyens techniques levés pour offrir un rapport investissement sur productivité bien plus favorable que l'offre moyenne sur le marché.

A cet effet, l'invention a pour objet une installation de traitement de déchets organiques selon la revendication 1 annexée.

Des caractéristiques avantageuses de cette installation font l'objet des revendications annexées 2 à 7.

L'invention a également pour objet un procédé de mise en œuvre d'une installation de traitement de déchets organiques telle que définie ci-dessus, selon la revendication 8 annexée.

Une caractéristique avantageuse de ce procédé fait l'objet de la revendication 9 annexée.

La présente invention concerne une installation et un procédé de digestion anaérobie multi-phases à chargement continu de type piston, à haute teneur en solides organiques volatils, à biomasse libre ou fixée et préférablement thermophile. D'une part, l'installation conforme à l'invention résulte d'une conception architecturale spécifique et, d'autre part, le procédé conforme à l'invention met en avant un mode particulier de transfert des substrats et fluides au sein du bioréacteur.

L'application d'une conception architecturale particulière a permis la différenciation de quatre cellules au sein d'un seul bioréacteur, chaque cellule étant un volume où se déroule majoritairement une des quatre principales phases de la biodigestion anaérobie. Les quatre cellules sont tout d'abord en interaction deux par deux du fait que la première abrite la seconde qui lui est concentrique, de même que la troisième abrite la quatrième qui lui est pareillement concentrique. De plus chaque couple de cellules concentriques comprend en son point bas un volume commun de transition et bioturbation horizontale au stade sédimentaire.

Le circuit de chauffage radiant est avantageusement réglé, selon la configuration, pour transporter un fluide caloporteur amené et maintenu selon les configurations thermophile ou mésophile.

Enfin le second couple de cellules comprend avantageusement une zone de bioturbation du point haut vers le point bas en dynamique sédimentaire. Les zones de bioturbations sont particulièrement riches en échanges biochimiques et bactériens.

Selon une caractéristique avantageuse de l'invention, le système pneumatique de brassage, micro bullage et circulation du substrat ne déstructure pas les biomes microbiologiques qui se forment spécifiquement au sein de chaque volume de phase mais les conserve dans une configuration de granulation spontanée. Ceci confère une efficacité remarquable au procédé conforme à l'invention.

L'installation et le procédé de traitement, conformes à l'invention, se démarquent clairement de l'enseignement de WO 2011/017420. Le système décrit dans ce document est un système biphasé, alors que l'installation et le procédé de l'invention en comptent quatre, physiquement séparées et séquentiellement unies dans un système piston. On y retrouve un premier anneau qui communique en fond de cuve avec une première cheminée, laquelle communique par une canalisation haute exclusivement avec le haut du deuxième anneau, lequel communique exclusivement en fond de cuve avec la deuxième cheminée 2, laquelle communique exclusivement en haut de cuve par une canalisation avec le dispositif d'essorage des digestats.

Dans le procédé de WO 2011/017420, du fait d'un brassage permanent et d'une architecture intérieure comportant des tubes ouverts, il n'est pas vraiment possible de déterminer si la première cuve est plus apte à favoriser l'hydrolyse ou l'acidogénèse, les compartiments intérieurs étant ouverts sur le haut et le bas, le volume de cette biocuve n'est pas exclusif à l'une ou l'autre de ces phase. Il en est de même dans la seconde cuve où acétoclastie et méthanogénèse sont indissociées. Dans ce procédé la séparation de phase est manifestement physico-chimique plutôt que biochimique au sens où le brassage par bullage et l'architecture intérieure des cuves semble faite pour séparer les matières en flottation des matières sédimentées, plutôt que pour réserver un temps de séjour biologique distinct et séquentiel aux substrats dans des volumes séparées au fil des quatre phases biochimiques d'un protocole de digestion anaérobie.

En résumé, le procédé selon WO 2011/017420 A2 s'organise sur une architecture tubulaire qui structure deux biocuves séparées, à l'intérieur de chacune desquelles sont installés trois tubes ouverts à leurs deux extrémités, donc sans séparation des substrats à l'intérieur de chaque biocuve. L'invention structure chacune de ses 2 biocuves en deux volumes, l'anneau et la cheminée, qui ne communiquent qu'en fond de biocuve dans une zone de bioturbation qui assure la transition de phase. L'invention organise donc strictement quatre volumes ayant chacun une capacité d'accueil différente et donc des durées de rétention biologique différentes. Dans le procédé de l'invention, le transfert des substrats du premier volume 1 successivement vers les deuxième, troisième et quatrième volumes, puis du quatrième volume vers la sortie du digesteur n'est possible que grâce au forçage pneumatique réalisé selon l'invention.

Le procédé de WO 2011/017420 A2 réalise un brassage gazeux permanent qui assimile son fonctionnement à celui mis en œuvre dans les digesteurs à contenu infiniment mélangé. Ainsi chacune des deux biocuves de WO 2011/017420 A2 est soumise à un mouvement convectif permanent des substrats, avec transfert par débordement d'un tube à l'autre à l'intérieur d'une même cuve. Dans ce procédé c'est l'ajout d'une dose de substrat en entrée de la première cuve qui détermine l'écoulement gravitaire par siphon de la première cuve à la seconde.

De manière totalement différente, le procédé de l'invention utilise les gaz de brassage pour créer une pression suffisante dans le ciel gazeux de l'anneau fermé dans sa partie haute, afin de faire monter par réaction dans la cheminée ouverte dans sa partie haute une quantité de substrat donnée qui correspond au volume de la dose de substrat primaire entrant dans le système. Cette surpression est obtenue quand le circuit de gaz circulé dans les biocuves est fermé et quand la canalisation qui relie le haut de la première cheminée à la surface (haut) du deuxième anneau est ouverte. A l'identique, quand il s'agit de procéder à l'évacuation des digestats la pression accumulée dans le ciel gazeux du deuxième anneau exerce une poussée sur le volume de substrat contenu dans cet anneau qui, par réaction, remonte dans la deuxième cheminée dont on a ouvert en sa partie haute la canalisation qui aboutit au dispositif d'essorage des digestats. A l'inverse du procédé WO 2011/017420 A2, selon l'invention il faut évacuer de la deuxième cheminée le volume correspondant à celui des matières entrantes, avant d'introduire les substrats entrants dans le premier anneau. En résumé le procédé de WO 2011/017420 A2 est un digesteur infiniment mélangé en deux phases avec séparation des matières flottantes et à alimentation continue gravitaire, alors que le procédé de l'invention est un digesteur à piston en quatre phases à alimentations semi continue avec forçage.

Le procédé de WO 2011/017420 A2 utilise un séparateur d'écume associé à des pompes à gaz de type tesla qui mobilisent les gaz de bullage lequel dispositif est aussi censé produire une évacuation partielle des matières en flottation dans les cuves. Le procédé de l'invention introduit, au contraire, du gaz anaérobie séparément dans la partie basse de l'anneau ou de la cheminée de chaque biocuve de manière à créer un micro-bullage à faible pression. Ce dernier va générer les conditions de bioturbation recherchées dans cette zone basse pour installer et maintenir les paramètres de mélange local à micro perturbation qui permettent la transition de phase biochimique. Dans le procédé selon l'invention, le bullage des effets mélangeurs intervient tout au long de la colonne montante. On peut aussi réaliser un brassage par pulsions ou spasmes en créant des conditions de pression dans les ciels gazeux des anneaux qui n'atteignent pas le point de transfert complet qui amènerait le substrat à déborder de la cheminée, et en relâchant rapidement cette pression pour créer un effet de reflux vertical qui favorise très significativement le brassage et le mélange des flottants par aspiration brusque dans la colonne descendante

Du fait de sa configuration, le procédé de WO 2011/017420 A2 est spécialement conçu pour des substrats liquides ou dilués alors que le procédé de l'invention se prête aussi parfaitement et particulièrement à des substrats à moyenne et haute teneur en solides totaux et même à haute viscosité. La gestion des températures annoncées pour ce procédé antérieur est donc de 75°C en cuve 1 et de 55°C en cuve 2. Le procédé de l'invention favorise, au contraire, avantageusement une population bactérienne particulière (pseudonomas) dans l'anneau 1 qui est maintenu typiquement à 40°C alors que la cheminée 1 qui lui est concentrique est maintenue typiquement à 35°C, de même l'anneau 2 est maintenu typiquement entre 50°C et 52 °C quand la cheminée 2 est amenée typiquement à 55°C. Cette régulation de température est automatisée dans le procédé de l'invention et est rendue possible par des transferts thermiques en bas de cuve réalisés avec des tubes immergés radiants séparés. De plus, particulièrement dans les cheminées, la gestion fine de la température et du flux de gaz de brassage permet la discrimination thermique recherchée.

### Description des figures

Les figures 1 à 4 illustrent des modes de réalisation de l'invention, mais ne limitent pas la portée de l'invention.
La figure 1 est une vue schématique, illustrant une installation de traitement de déchets conforme à l'invention ;
La figure 2 est une vue de face, illustrant à plus grande échelle deux cuves de traitement appartenant à l'installation de la figure 1 ;
La figure 3 est une vue de dessus, illustrant ces deux cuves de traitement.
La figure 4 est une vue en coupe de ces deux cuves.

Les références numériques suivantes sont utilisées dans la présente description:

| | | | |
|---|---|---|---|
| 1 | Première cuve | 2 | Deuxième cuve |
| 11 | Cheminée | 21 | Cheminée |
| 12 | Anneau | 22 | Anneau |
| 13 | Fond | 23 | Fond |
| 14 | Périphérie couvercle | 24 | Périphérie couvercle |
| 14' | Centre couvercle | 24' | Centre couvercle |
| 15 | Paroi de séparation | 25 | Paroi de séparation |
| 15' | Echancrures | 25' | Echancrures |
| 16 | Zone commune | 26 | Zone commune |
| 17 | Dispositif de brassage | 27 | Dispositif de brassage |
| 17' | Dispositif micro-bullage | 27' | Dispositif micro-bullage |
| 19 | Enveloppe externe | 29 | Enveloppe externe |
| E1 | Entrée substrats | T | Ligne transfert |
| ALIM | Ligne alimentation | E2 | Entrée substrats |
| S1 | Sortie substrats | S2 | Sortie substrats |
| EVAC | Evacuation substrats | 31,31' | Sortie biogaz |
| 43,44 | Entrée gaz circulation | 47,48 | Sortie gaz brassage |
| 3 | Vis essoreuse | 32 | Sortie digestats |
| 33 | Sortie éluats | 4 | Ballon de pression |
| 41,42 | Sorties de 4 | 45,46 | Lignes de 4 vers 2 |
| 5 | Dispositif de filtration | 51 | Sortie de 5 |
| 52,53 | Lignes de 5 vers 1 | BIO | Zone de bioturbation |

### Description détaillée

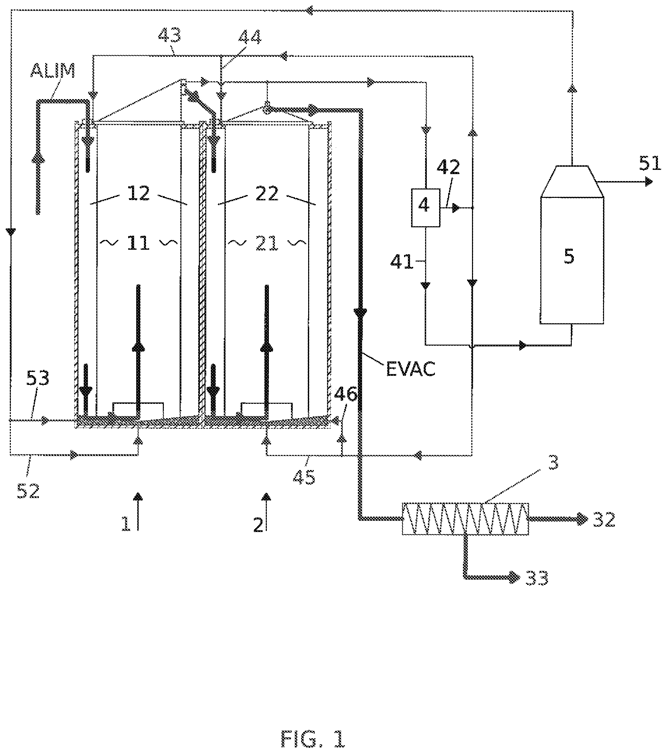

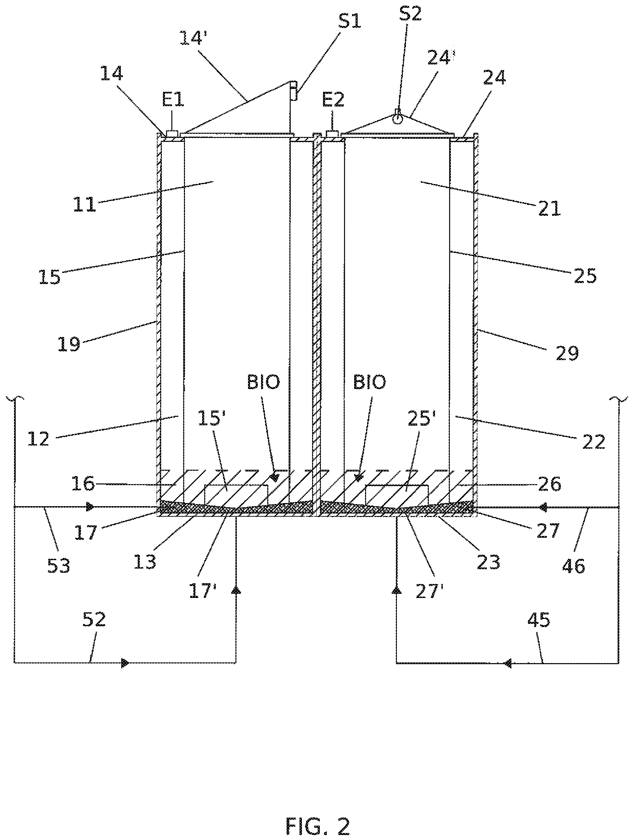

L'installation de traitement de déchets, conforme à l'invention, est illustrée dans sa généralité sur la figure 1. Elle comprend tout d'abord deux cuves de traitement 1 et 2, qui vont être décrites plus en détail en référence à la figure 2.

Les cuves 1 et 2, qui sont globalement identiques, présentent dans l'exemple illustré une section rectangulaire. A titre de variante, on peut prévoir qu'elles présentent d'autres formes, notamment des parallélépipèdes de section différente, ou bien encore des cylindres de section circulaire ou pseudo cylindre de section elliptique. Les dimensions des cuves sont adaptées en fonction de la nature et de la quantité du flux de substrats à traiter.

Chaque cuve 1 ou 2 définit une cellule (ou compartiment) centrale 11 ou 21, encore dénommée cheminée. Cette dernière est entourée par une cellule (ou compartiment) périphérique annulaire 12 ou 22, encore dénommée anneau. Chaque cheminée et chaque anneau sont mutuellement concentriques, dans l'exemple illustré.

Chaque cuve 1 ou 2 comprend un fond 13 ou 23, un couvercle 14,14' ou 24, 24', ainsi qu'une enveloppe externe 19 ou 29 reliant ce fond et ce couvercle. Cette enveloppe, qui forme la paroi périphérique d'un anneau respectif, est composée de quatre éléments mécaniques à savoir, de l'extérieur vers l'intérieur :
- une peau externe verticale associée au fond précité, pour assurer la fonction d'exosquelette ;
- une couche d'isolant qui peut être apposée sur les faces externes ou internes de cette peau ;
- un circuit de préchauffage qui est disposé sur les faces internes de cette peau et en fond de cellule ;
- un cuvelage étanche qui peut être constitué soit par la face interne de la peau externe elle même si son matériau s'y prête, soit une peinture ou un enduit étanche soit par un film ou un assemblage soudé de plaques en plastique ;
- un rebord d'assemblage par flasque mâle, qui s'étend sur le périmètre haut de la peau externe pour coopérer avec un rebord d'assemblage d'un flasque femelle, qui s'étend sur le périmètre bas du couvercle ;
- un joint d'étanchéité par compression qui s'intercale entre le flasque de la peau externe et celui du couvercle.

Chaque couvercle recouvre le volume total de la cuve, mais différencie strictement le volume de chaque anneau par rapport à celui de la cheminée. A cet effet, une paroi de séparation 15 ou 25 s'étend à partir de ce couvercle, en direction du fond. Il est à noter que chaque paroi 15 ou 25 ne s'étend pas sur la totalité de la hauteur de la cuve, de manière à délimiter une zone dite commune 16 ou 26, au voisinage du fond. Dans l'exemple illustré, chaque paroi 15 ou 25 est creusée d'échancrures 15' ou 25', afin d'assurer cette communication entre un anneau et une cheminée respectifs. Il est important de noter que l'on fait varier la configuration aisément les zones d'échancrure pour dimensionner la dynamique des communications entre un anneau et une cheminée.

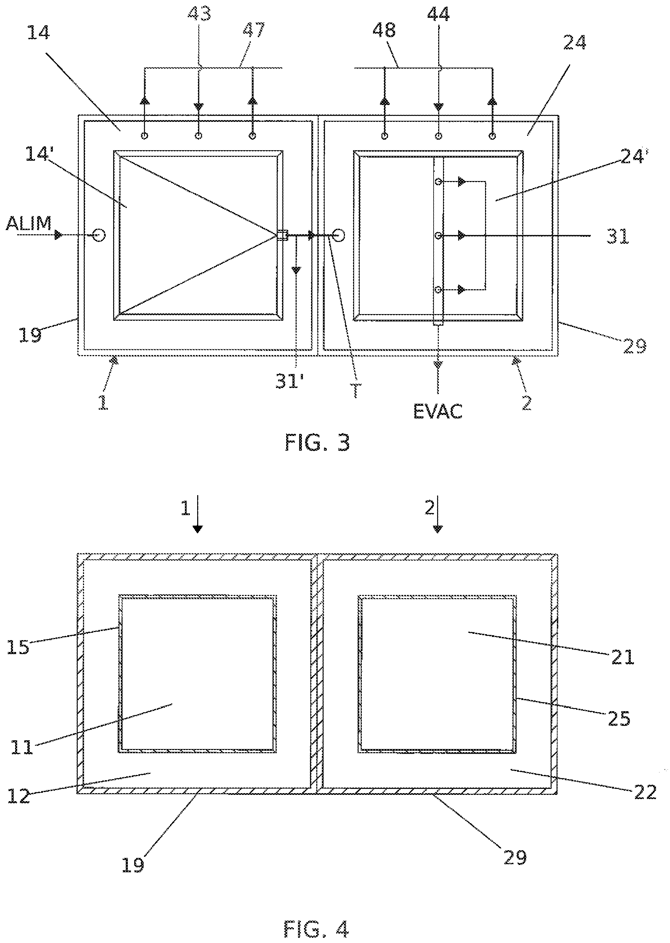

Les deux couvercles 14, 14' ou 24, 24' sont identiques au sens où chacun d'eux est composé de deux parties concentriques, dont la première 14 ou 24 recouvre un anneau respectif 12 ou 22, et dont la seconde 14' ou 24' recouvre une cheminée respective 11 ou 21. Dans tous les cas ce couvercle scelle hermétiquement le volume intérieur d'une cuve respective. Ces deux couvercles sont en revanche fonctionnellement différents, comme le montrent notamment les figures 2 et 3.

En effet, la partie périphérique 14 du premier couvercle possède une entrée E1 (figure 2) de substrats bruts, via une ligne d'alimentation ALIM (figure 3). De plus, la partie centrale 14' de ce premier couvercle possède une sortie S1 (figure 2) pour assurer le transfert de la première cheminée vers le deuxième anneau, via une ligne de transfert T (figure 3). En revanche la partie périphérique 24 du second couvercle possède une entrée E2 des substrats partiellement biodigérés, via la ligne précitée T, On notera que la sortie S1 est surélevée par rapport à l'entrée E2, afin de favoriser un transfert par gravité des substrats entre les deux cuves 1 et 2.

Enfin la partie centrale 24' de ce second couvercle possède une sortie S2 (figure 2) pour assurer l'évacuation des substrats hors de la deuxième cheminée, via une ligne EVAC, vers la zone de traitement postérieur de l'installation, notamment l'essorage des digestats. Cette sortie S2 est également surélevée, afin de favoriser une évacuation par gravité des substrats.

Deux conduites 31 et 31' permettent par ailleurs l'évacuation, hors des deux cuves, du biogaz et du gaz de micro-bullage qui sera décrit ci-dessous. Par ailleurs la partie périphérique de chaque couvercle est alimentée, via des lignes 43 et 44, en gaz de circulation apte à faire circuler les déchets à l'intérieur des cuves. Cette partie périphérique possède aussi des sorties des gaz de brassage communiquant, via des lignes 47 et 48, avec un dispositif de filtration ou de recirculation du biogaz qui sera également décrit ci-dessous.

Chacune des deux cuves 1 ou 2 est munie, vers son fond, d'un double fond étanche, qui est légèrement concave. Ce double fond renferme un dispositif de brassage 17 ou 27, composé d'une série de buses, ainsi qu'un dispositif de micro-bullage 17' ou 27', composé de disques céramiques micro-perforés. Ces buses et ces disques sont de type connu en soit de sorte que, sur la figure 2, ils sont représentés de façon schématique.

L'installation conforme à l'invention comprend en outre différents organes mécaniques, de type classique, dont certains sont représentés sur la figure 1.

On retrouve tout d'abord un système d'essorage des substrats en sortie de la deuxième cheminée, réalisée dans l'exemple illustré sous la forme d'une vis essoreuse 3. A titre de variante, on peut prévoir une centrifugeuse, ou encore une simple enceinte de décantation. Cette vis 3 est reliée à la deuxième cheminée par la ligne d'évacuation EVAC précitée, apte à véhiculer les substrats sortant de la cuve 2. Cette vis possède deux sorties, à savoir une sortie 32 pour les digestats et une sortie 33 pour les éluats. Dans le cas d'une application du procédé à des intrants de très faible teneur en solides, dans une configuration à biomasse fixée, il est courant que ce dispositif d'essorage soit évité ou remplacé par un système de filtration tangentiel ou traversant.

On retrouve en outre un réservoir, ou ballon de pression 4, qui est relié aux cheminées par les conduites 31 et 31' ci-dessus, aptes à véhiculer le biogaz sortant de la cuve 2. Ce ballon 4 possède deux sorties, dont l'une 41 communique avec un dispositif de filtration 5, qui sera décrit plus en détail ci-dessous.

Par ailleurs, plusieurs lignes de recyclage du biogaz s'étendent à partir de l'autre sortie 42 du ballon 4. On retrouve :
- la ligne précitée 43 qui débouche en partie supérieure de l'anneau 12,
- la ligne précitée 44 qui débouche en partie supérieure de l'anneau 22,
- une ligne 45 qui débouche au fond de la cheminée 21 et alimente le dispositif de micro-bullage 27,
- ainsi qu'une ligne 46 qui alimente des buses de brassage prévues sur la paroi latérale externe de la cuve 2.

Le dispositif 5 de filtration du biogaz a pour fonction de séparer le CO₂ du méthane CH₄. Il peut prendre la forme d'une cellule de solubilisation à l'eau, ou tout autre dispositif équivalent. Ce dispositif 5 est typiquement alimenté en dépression par une pompe aéraulique de classe ATEX. Il possède une ligne de sortie 51 pour le méthane filtré, ainsi que deux lignes de recyclage :
- une ligne 52 qui débouche au fond de la cheminée 11 et alimente le dispositif de micro-bullage 17
- ainsi qu'une ligne 53 qui alimente des buses de brassage non représentées, prévues sur la paroi latérale externe de la cuve 1.

La mise en œuvre de l'installation décrite ci-dessus, va maintenant être explicitée dans ce qui suit. On notera que l'installation comprend, outre les organes mécaniques illustrés sur les figures, des organes supplémentaires qui ne sont pas représentés. Ils sont de type classique et ne font pas partie de l'invention, de sorte qu'ils sont décrits succinctement dans le cadre de la mise en œuvre précitée.

Avant d'être introduits dans les cuves de traitement, les déchets font l'objet d'un broyage de type classique, afin de réduire leur taille relative dans une granulométrie n'excédant pas 25 mm. Ce broyage peut être réalisé dans un dispositif de type broyeur lent à couteau à double axe, alimenté par une trémie de chargement. Cette dernière est typiquement munie d'un couvercle assurant la protection de l'opérateur et d'une bascule hydraulique qui permet le levage et la vidange des poubelles (ISO) de 120 litres ou 240 litres.

En aval du broyeur précité, les déchets (encore appelés « substrat ») sont admis et contenus dans un système de préchauffage et de mélange, constitué par exemple d'une cuve chargée par voie gravitaire avec ces déchets broyés. Cette cuve peut recevoir un liquide de dilution amené typiquement à 55°C, constitué d'un mélange d'éluats, formés en aval du procédé, et d'eau. Cette cuve est typiquement équipée d'un réseau tubulaire où circule un flux caloporteur assurant le chauffage définitif du substrat dilué préchauffé pour que l'ensemble atteigne la température cible de 55°C

En appui du préchauffage des substrats introduits et de l'isolation des cuves, le circuit de réchauffage est amené à une température supérieure de quelques degrés à celle ciblée, soit 58°C ou 38°C, jusqu'à ce que les températures cibles mesurées par des capteurs au sein des cellules soient atteintes, ce qui provoque l'arrêt de la circulation du fluide. Par ailleurs, dès que les capteurs renvoient un indice de baisse de température, le fluide caloporteur est alors immédiatement réchauffé et recirculé pour contrer l'inertie du système.

On peut également prévoir une pompe de relevage, acceptant avantageusement des flux hautement turbides avec une granulométrie maximum de 35 mm. Cette pompe est destinée à alimenter le bioréacteur, en partie haute du premier anneau, avec le substrat.

De manière générale, les substrats sont admis par ligne ALIM, progressent vers le bas le long du premier anneau sous l'effet du biogaz admis par la ligne 43. Dans cette enceinte ils se prêtent à une première étape de digestion par bio-oxydation anaérobie, à savoir une hydrolyse. Cette première transformation est rendue possible du fait de la présence particulièrement dense de bactéries anaérobies hydrolytiques dans le premier anneau. Durant leur passage dans cet anneau, le gaz soufflé depuis la ligne 53, grâce aux buses 17, génère un effet turbulent du bas vers le haut sans pour autant déstructurer les biomes bactériens granuleux qui s'y installent et assure ainsi une fonction de brassage et prévient la formation d'une croûte en surface lors du passage du gaz dans le volume non immergé de l'anneau.

Puis les substrats sensiblement totalement hydrolysés progressent vers le haut le long de la première cheminée 11, sous l'effet conjoint du biogaz admis par la ligne 43, et du gaz soufflé par la ligne 52. Le réseau de disques céramiques micro-perforés 17, alimenté par cette ligne 52, délivre un flux de micro-bulles à effet de brassage mais surtout de flottation des sédiments. Ces alimentations séparées permettent de gérer distinctement le décompactage et le brassage dans les anneaux et la flottation dans les cheminées en dehors ou en simultané des phases de remplissage et transfert de substrats.

Le dispositif de brassage et flottation fonctionne en circuit fermé afin d'éviter d'une part le rejet de gaz et pour prévenir les risques de surpression en haut de cuve. On notera que ce circuit fermé tend même à créer une mise en dépression de la cheminée, en particulier dans la partie haute de cette dernière, ce qui favorise la remontée du substrat à l'intérieur de cette cheminée. Durant leur séjour dans cette cheminée, les substrats subissent une deuxième étape de digestion, à savoir une acidogénèse, du fait de la présence massive de bactéries anaérobies acidophiles dans cette enceinte.

Les substrats partiellement digérés sont ensuite transférés, via la ligne T, vers la deuxième cuve 2. Ils progressent vers le bas le long du deuxième anneau sous l'effet du biogaz admis par la ligne 44. Ils subissent alors une troisième étape de digestion, à savoir une acétogénèse du fait de la présence massive de bactéries anaérobies acétoclastes dans cette enceinte Durant leur passage dans cet anneau, le gaz soufflé depuis la ligne 46, grâce aux buses précitées, génère un effet turbulent et assure une fonction de brassage à l'identique de ce qui advient dans l'anneau 1.

Puis les substrats progressent vers le haut le long de la deuxième cheminée 21, sous l'effet conjoint du biogaz ou du CO₂ admis d'une part via la ligne 44 et, d'autre part, via la ligne 45. Comme décrit ci-dessus dans le cas de la première cuve, il se produit un effet de brassage mais surtout de flottation des sédiments. Durant leur séjour dans cette cheminée, les substrats subissent l'étape finale de digestion anaérobie, la méthanogénèse qui est intimement liée à la phase précédente acétoclaste et qui est optimisée du fait de la présence massive de bactéries anaérobies méthanogènes dans cette enceinte.

Conformément à l'invention, au-delà de la séparation des phases, une transition biochimique continue est assurée au sein de la zone de bioturbation formée en bas de chaque cuve, du fait de la permanence d'un volume de mélange des substrats au point bas des anneaux jusqu'à la hauteur des échancrures de la cheminée qui y est installée. Cette zone de bioturbation est affectée de la référence BIO sur la figure 2. On notera aussi l'existence d'une zone de bioturbation en dynamique sédimentaire, à savoir en d'autres termes gravitaire, opérée du point haut du deuxième anneau vers la zone basse de la deuxième cuve.

L'injection de flux de gaz à intervalles réguliers mais brefs tant au niveau des anneaux que des cheminées, en dehors des phases de remplissage et transfert et pendant les phases de transfert produit un brassage et un décompactage non destructif des biomes microbiologiques induisant une granulation des solides sédimentés. L'injection de flux de gaz dans les cheminées, durant les phases de remplissage et transfert et en dehors des phases de transfert autant que de besoin, entraîne la remontée par flottation des sédiments et favorise leur transport vers le haut dans la dynamique de transfert.

De manière générale le gaz, à savoir le biogaz et/ou le CO₂, qui est injecté/recyclé dans les cuves, assure trois fonctions hydrauliques et aérauliques principales: circulation, brassage, micro-bullage et une fonction biochimique en apportant soit de l'hydrogène (H₂S) associable soit du carbone (CO₂). L'homme du métier ajustera notamment les conditions opératoires et le déroulement de chacune de ces trois étapes, en fonction des paramètres du substrat et de la spéciation bactérienne en fonction des quatre phases différenciés au sein des quatre enceintes du biodigesteur. Les conditions opératoires sont notamment le choix d'injecter du biogaz brut ou plus ou moins épuré en CO₂ ou H₂S ou du CO₂ uniquement. Il adaptera aussi les débits de gaz respectifs et les diamètres de bulles respectifs en vue de remplir chaque fonction. Le déroulement des étapes comprend notamment leur durée, leur fréquence, la concomitance éventuelle entre ces étapes. Les paramètres du substrat comprennent notamment leur nature, leur turbidité, leur coalescence, ainsi que leur débit.

Les substrats digérés de façon sensiblement complète sont alors évacués, via la ligne EVAC, vers la vis 3. Ils sont alors séparés, de façon connue en soi, en une fraction sensiblement solide ou digestats, extraite via la ligne 33, et une fraction sensiblement liquide ou éluats, via la ligne 32.

Par ailleurs, du biogaz est évacué en direction du ballon 4, via la ligne 31. Une première partie de ce biogaz est recyclé en direction des cuves 1 et 2, via les lignes 43 à 46. L'autre partie de ce biogaz est dirigé, via la ligne 41, vers le dispositif de filtration 5, où il est séparé en une fraction riche en méthane évacuée par la ligne 51, ainsi qu'une fraction riche en CO₂ recyclée par les lignes 52 et 53.

On notera que, dans l'exemple illustré, du biogaz et du CO₂ sont recyclés en direction des cuves. A titre de variante, seul le biogaz ou seul le CO₂ peuvent être recyclés. A titre de variante supplémentaire, à la fois le biogaz et le CO₂ peuvent être recyclés, mais en des endroits des cuves qui sont différents de ceux illustrés sur les figures.

De manière plus précise, on suppose que l'on se trouve dans la phase initiale de la mise en œuvre, qui correspond à la toute première introduction de substrats dans la première cuve 1. La fraction de déchets ainsi introduite séjourne d'abord dans l'anneau de cette première cuve puis, sous l'effet du gaz de circulation, est transféré vers la cheminée de la première cuve. Une fois remplie, la cheminée 1 opère un transfert vers l'anneau 2 de la cuve 2 pour un volume équivalent à celui des substrats bruts introduits dans l'anneau 1. Le remplissage de l'anneau 2 et de la cheminée 2 se poursuivent au rythme de l'introduction de substrats bruts en amont dans le système. Dès lors que la cheminée 2 est remplie on suppose désormais que l'on se trouve en régime « normal » de mise en œuvre. On voit bien que dans ce régime normal le procédé conforme à l'invention est de type continu discrétisé, à savoir qu'il s'oppose à un traitement de type « lot » ou « batch », mais qu'il n'est généralement pas rigoureusement continu encore qu'il pourrait l'être dans le cas de production de substrats issus par exemple d'un procédé de type agro-industriel lui-même continu.

Dans cette configuration « normale », le travail est effectué en deux temps :
- Le deuxième anneau reçoit en partie haute une injection de biogaz enrichi en biogaz ou CO₂ sous pression. Étant étanche il se comporte alors comme une chambre de pression d'où s'opère un transfert des fluides non compressibles à savoir la montée des substrats contenus dans la deuxième cheminée. Cette dernière, qui est ouverte vers l'extérieur en direction de la vis d'essorage, se comporte alors comme un conduit d'expansion.
- Une fois qu'un volume de substrat sortant, qui est équivalent à celui du substrat à introduire dans le bioréacteur, a été évacué de la deuxième cuve selon l'étape décrite ci-dessus, on injecte du biogaz ou du CO₂ sous une pression appropriée dans le premier anneau, pour qu'il transfère le substrat dans la première cheminée. Étant donné que cette dernière est ouverte en son point haut vers le deuxième anneau, le substrat est transféré, en fin de course d'expansion, vers le point haut du deuxième anneau via la ligne T.
- En même temps que du biogaz ou du CO₂ est injecté dans le deuxième anneau, un micro bullage intensif mais bref est effectué au pied de la deuxième cheminée. Cela génère un entraînement des sédiments vers le haut lors du transfert d'une dose dans le système d'expansion au long de la cheminée. Un micro bullage analogue est réalisé dans la première cuve, au moment où le biogaz est injecté sous pression au point haut de l'anneau 1.

Un réseau de capteurs non représentés mesurent en temps réel ou légèrement différé les valeurs obtenues pour différents paramètres, tels que : la température, le pH, la turbidité des substrats au cours des différentes phases, la composition chimique, la température et l'humidité relative du biogaz et du biométhane épuré mais aussi le niveau des substrats dans les différentes cuves.

Un jeu de plusieurs automates industriels programmables non représentés traitent les signaux reçus des capteurs, infèrent le comportement d'effecteurs et rendent compte de l'état du système sur un poste de contrôle distant.

Un réseau d'effecteurs non représentés tels que des électrovannes hydrauliques ou pneumatiques régulent la circulation des flux de substrats, digestats, éluats commandés par les automates programmables ou directement par l'opérateur humain.

Un réseau hydraulique non représenté transporte des éluats pour servir de fluide de dé-coagulation aux points critiques des canalisations de transferts de substrats.

Une fraction de la boucle du circuit de réchauffage, non représentée, traverse un échangeur thermique qui est sourcé par le dispositif de refroidissement du groupe électrogène et transmet ainsi des calories au fluide caloporteur circulant dans le circuit de réchauffage de l'installation.

L'invention n'est pas limitée aux exemples décrits et représentés, et n'est limitée que par les revendications.

Les caractéristiques dimensionnelles des différents constituants de l'installation sont déterminées d'abord par un ensemble de facteurs physico-chimiques qui influent sur le volume des cuves, sur leur diamètre mais surtout sur leur hauteur. Ces contraintes relèvent donc de la faisabilité technique du procédé.

Par ailleurs des facteurs économiques et contextuels peuvent aussi influencer le dimensionnement par effet de contraintes budgétaires, environnementales, ou culturelles.

Considérant que les transferts de substrats résultent d'une poussée du haut vers le bas exercée par un gaz mis en pression dans une chambre délimitée par la face intérieure du couvercle au niveau de l'anneau et la surface du substrat contenu dans l'anneau, que cette pression de gaz induit une surpression des gaz dissous dans les substrats, que l'hydrogène à une pression de 2 bars inhibe une partie significative des réactions biochimique de la séquence des quatre phases de la digestion méthanique, nous avons déterminé une limite à la taille des cellules sur deux grandeurs, la hauteur et le volume de l'anneau, en considérant un substrat très turbide titrant jusqu'à 55 % de solides totaux.

En effet plus le volume à transférer est élevé et plus le substrat est turbide, plus l'effort de poussée doit être important pour vaincre à la fois la masse et les frottements qui génèrent des pertes de charge.

La grande variabilité des configurations réalisables rend quasiment impossible de modéliser les valeurs limites avec un degré de précision élevé parce qu'il faut prendre en compte la largeur de l'anneau, sa hauteur, le type de substrat et les paramètres déterminant les forces de frottement sur des configurations régulières et singulières créant des perte de charge. Nous avons donc eu recours à une approche expérimentale sommaire en prototype pour déterminer des valeurs limites qui ne délivrent pas le champ holistique de faisabilité du procédé mais seulement des seuils que nous avons adoptés pour la sécurité qu'ils procurent dans une zone de faisabilité certaine.

Ces seuils dimensionnels se traduisent avantageusement par les valeurs typiques suivantes, données à titre non limitatif:
- Hauteur maximale du substrat dans les cuves : entre 7 mètres et 10 mètres.
- Largeur maximum de l'anneau : entre 1,5 mètres et 2,5 mètres.
- Diamètre maximal de la cheminée : entre 3 mètres et 4 mètres.
- Turbidité maximale du substrat : entre 55 % et 75 % de solides totaux en suspension.

Par ailleurs les inventeurs ont considéré le besoin d'optimiser la thermodynamique du système qui est thermophile (55°C) en considérant le fait qu'au-delà d'un volume d'environ 500 m³ les transferts de chaleur et le brassage aéraulique dépassaient le seuil d'acceptabilité de consommation énergétique que nous avons fixé à 25 % de la capacité de production tirée de la valorisation du biométhane.

A titre d'exemple purement non limitatif, pour produire une offre commerciale adapté aux cibles visé par l'inventeur, celui-ci a décliné l'installation qui vient d'être décrite en plusieurs modèles qui varient en fonction de leur capacité de traitement et du type de substrat à traiter. L'un de ces modèles est configuré pour être abrité dans deux conteneurs maritimes de type « 20' high Cube », au sens de la norme ISO de 1967, lesquels sont disposés à plat. Le premier conteneur contient les bioréacteurs, la trémie d'alimentation associée au broyeur et à la cuve de dilution et préchauffage, ainsi qu'une pompe d'alimentation. Le second conteneur est réservé à l'armoire électrique, au poste de commande, à la vis extrudeuse, aux groupes électrogènes et aux filtres pour le biogaz.

Cet équipement extrêmement compact et versatile est aussi modulaire puisqu'il est possible d'ajouter jusqu'à trois « conteneurs bioréacteur » pour augmenter les capacités de traitement de la station selon ses besoins du moment, sans que le conteneur technique ne soit sous dimensionné. Avec une telle installation un opérateur est capable de traiter chaque jour jusqu'à 1 m³ de substrat broyé et dilué, ce qui représente par exemple environ 375 kg de déchets de cuisine et de service et 25 kg de graisses issues de bac à graisse. Dans cette configuration la production de biométhane sera d'environ 34,5 Nm³ par jour, les digestats liquides ou éluats sont produits à hauteur de 0,14 m³ par jour et les digestats solides après extrusion à hauteur d'environ 0,35 m³ par jour.

## Revendications

1. Installation de traitement de déchets organiques, pour produire du biogaz et récupérer au moins une partie du biogaz produit, cette installation comprenant :
- une première cuve (1) comportant des premiers moyens de séparation (15) s'étendant sur une partie de la hauteur de la cuve, de manière à définir un premier compartiment central, ou cheminée (11), un premier compartiment périphérique, ou anneau (12), fermé dans sa partie haute, ainsi qu'un premier compartiment de brassage et d'échanges biochimiques (16) assurant exclusivement la communication entre ces deux compartiments, en partie basse de la cuve, le premier compartiment de brassage comprenant des premiers moyens de brassage (17),
- une deuxième cuve (2) comportant des deuxièmes moyens de séparation (25) s'étendant sur une partie de la hauteur de la cuve, de manière à définir un deuxième compartiment central, ou cheminée (21), un deuxième compartiment périphérique, ou anneau (22), fermé dans sa partie haute, ainsi qu'un deuxième compartiment de brassage et d'échanges biochimiques (26) assurant exclusivement la communication entre ces deux compartiments, en partie basse de la cuve, le deuxième compartiment de brassage comprenant des deuxièmes moyens de brassage (27),
- des moyens d'arrivée (ALIM) des déchets à traiter, dans le premier anneau,
- des moyens de transfert (T) des déchets partiellement traités, depuis la première cheminée (11) vers le deuxième anneau, de telle sorte que la première cheminée (11) communique en partie haute exclusivement avec le haut du deuxième anneau (22), par une canalisation,
- des moyens d'évacuation (EVAC) des déchets traités, hors de la deuxième cheminée,
- des moyens (43, 44) de circulation des déchets par forçage pneumatique, depuis le premier anneau (12) vers la deuxième cheminée (21), ces moyens de circulation comprenant :
∘ une ligne (43) de gaz de circulation débouchant en partie supérieure du premier anneau (12) et permettant de créer une pression suffisante dans le ciel gazeux de l'anneau (12) afin de faire monter dans la première cheminée (11) par forçage pneumatique un volume de substrat correspondant à celui des matières entrantes, et
∘ une ligne (44) de gaz de circulation débouchant en partie supérieure du deuxième anneau (22) permettant de même de procéder par forçage pneumatique à l'évacuation de la deuxième cheminée (21) d'un volume de digestats correspondant à celui des matières entrantes ;
où l'installation est donc agencée de telle sorte que les déchets à traiter sont transférés successivement depuis le premier volume formé par le premier anneau (12), vers un deuxième volume formé par la première cheminée (11), puis vers un troisième volume formé par le deuxième anneau (22), puis vers un quatrième volume formé par la deuxième cheminée (21), avant d'être évacués de la deuxième cheminée (21) par une canalisation en partie haute de la deuxième cuve (2), grâce à une alimentation semi-continue avec forçage pneumatique.

2. Installation selon la revendication 1, **caractérisée en ce que** les moyens de transfert des déchets partiellement traités comprennent ladite canalisation, laquelle forme une ligne de transfert reliant une sortie de la première cuve et une entrée de la deuxième cuve, ladite sortie étant à une altitude supérieure à celle de ladite entrée.

3. Installation selon l'une quelconque des revendications précédentes, **caractérisée en ce qu'**elle comprend en outre des moyens de flottation (52, 17 ; 45, 27) des sédiments, prévus au voisinage du fond d'au moins une cheminée.

4. Installation selon la revendication précédente, **caractérisée en ce que** les moyens de flottation des sédiments comprennent au moins une conduite destinée à acheminer un gaz de micro-bullage, avantageusement un gaz anaérobie tel un biogaz, de l'azote ou du dioxyde de carbone, cette conduite alimentant au moins un dispositif de micro-bullage prévu au voisinage du fond d'une cheminée respective.

5. Installation selon l'une quelconque des revendications précédentes, **caractérisée en ce que**, dans sa configuration thermophile ou mésophile, l'installation comprend des moyens de chauffage des cuves.

6. Installation selon la revendication précédente, **caractérisée en ce que** les moyens de chauffage comprennent au moins un réseau de canalisations radiantes disposées sur les parois internes des anneaux et/ou sur le fond de ces anneaux et/ou sur le fond des cheminées.

7. Installation selon l'une quelconque des revendications précédentes, **caractérisée en ce qu'**elle comprend une unique première cuve (1) comportant un unique compartiment central (11) et un unique compartiment périphérique (12), ainsi qu'une unique deuxième cuve (2) comportant un unique compartiment central (21) et un unique compartiment périphérique (22).

8. Procédé de mise en œuvre d'une installation de traitement de déchets organiques selon l'une des revendications précédentes, ce procédé comprenant les étapes suivantes :
- on admet des déchets à traiter dans la première cuve (1),
- on réalise au moins une partie d'une étape d'hydrolyse dans le premier anneau (12) et au moins une partie d'une étape d'acidogénèse dans la première cheminée (11),
- on transfère les déchets partiellement traités depuis la première cheminée vers le deuxième anneau (22),
- on réalise au moins une partie d'une étape d'acétogénèse dans le deuxième anneau (22) et au moins une partie d'une étape de méthanogénèse dans la deuxième cheminée (21),
- on évacue les déchets traités, hors de la deuxième cheminée,
- procédé dans lequel on active les moyens de circulation (43, 44), de telle sorte que les déchets à traiter sont transférés successivement depuis le premier volume formé par le premier anneau (12), vers un deuxième volume formé par la première cheminée (11), puis vers un troisième volume formé par le deuxième anneau (22), puis vers un quatrième volume formé par la deuxième cheminée (21), avant d'être évacués de la deuxième cheminée (21) par une canalisation en partie haute de la deuxième cuve (2), grâce à une alimentation semi-continue avec forçage pneumatique,
- et procédé dans lequel en outre on évacue une fraction de déchets présents dans la deuxième cuve, puis on admet une autre fraction de déchets dans la première cuve, la fraction évacuée et la fraction admise ayant sensiblement les mêmes volumes.

9. Procédé selon la revendication 8, **caractérisé en ce qu'**on affecte, pour chaque cuve (1, 2), des capacités d'accueil différentes et donc des durées de rétention biologique différentes au compartiment central (11, 21) et au compartiment périphérique (12, 22).

## Patentansprüche

1. Anlage zur Behandlung von biologischen Abfällen zur Herstellung von Biogas und Rückgewinnung mindestens eines Teils des hergestellten Biogases, wobei diese Anlage umfasst:
- einen ersten Behälter (1), der erste Trennmittel (15) beinhaltet, die sich über einen Teil der Höhe des Behälters erstrecken, um ein erstes zentrales Fach, oder Kamin (11), ein erstes in seinem oberen Teil geschlossenes peripheres Fach, oder Ring (12), sowie ein erstes Fach zum Mischen und für biochemische Austausche (16) zu definieren, welches ausschließlich für die Kommunikation zwischen den Fächern sorgt, im unteren Teil des Behälters, wobei das erste Fach zum Mischen erste Mischmittel (17) umfasst,
- einen zweiten Behälter (2) der zweite Trennmittel (25) beinhaltet, die sich über einen Teil der Höhe des Behälters erstrecken, um ein zweites zentrales Fach, oder Kamin (21), ein zweites in seinem oberen Teil geschlossenes peripheres Fach, oder Ring (22), sowie ein zweites Fach zum Mischen und für biochemische Austausche (26) zu definieren, welches ausschließlich für die Kommunikation zwischen den Fächern sorgt, im unteren Teil des Behälters, wobei das zweite Fach zum Mischen zweite Mischmittel (27) umfasst,
- Mittel zum Zuführen (ALIM) der zu behandelnden Abfälle im ersten Ring,
- Mittel für den Transfer (T) der teilweise behandelten Abfälle vom ersten Kamin (11) zum zweiten Ring, derart, dass der erste Kamin (11) im oberen Teil ausschließlich mit der Oberseite des zweiten Ringes (22) über eine Kanalisation kommuniziert,
- Mittel zum Austragen (EVAC) der behandelten Abfälle aus dem zweiten Kamin,
- Mittel (43, 44) zum Zirkulieren der Abfälle durch pneumatisches Forcieren aus dem ersten Ring (12) zum zweiten Kamin (21), wobei diese Mittel zum Zirkulieren Folgendes umfassen:
° eine Gaszirkulationsleitung (43), die in den oberen Teil des ersten Ringes (12) mündet, und es ermöglicht, einen ausreichenden Druck in der Gaszusammensetzung des Ringes (12) zu schaffen, um im ersten Kamin (11) durch pneumatisches Forcieren ein Substratvolumen ansteigen zu lassen, das jenem der eintretenden Materialien entspricht, und
° eine Gaszirkulationsleitung (44), die in den oberen Teil des zweiten Ringes (22) mündet, die es ebenso ermöglicht, durch pneumatisches Forcieren das Austragen aus dem zweiten Kamin (21) eines Gärrestvolumens vorzunehmen, das jenem der eintretenden Materialien entspricht;
wobei die Anlage somit derart ausgeführt ist, dass die zu behandelnden Abfälle nacheinander aus dem ersten Volumen, das durch den ersten Ring (12) gebildet wird, in ein zweites Volumen, das durch den ersten Kamin (11) gebildet wird, und danach in ein drittes Volumen, das durch den zweiten Ring (22) gebildet wird, danach in ein viertes Volumen, das durch den zweiten Kamin (21) gebildet wird, transferiert werden, bevor sie aus dem zweiten Kamin (21) durch eine Kanalisation ausgehend von dem zweiten Behälter (2) dank einer halbkontinuierlichen Zuführung mit pneumatischer Forcierung ausgetragen werden.

2. Anlage nach Anspruch 1, **dadurch gekennzeichnet, dass** die Mittel für den Transfer der teilweise behandelten Abfälle die Kanalisation umfassen, welche eine Transferleitung bildet, die einen Ausgang des ersten Behälters und einen Eingang des zweiten Behälters verbindet, wobei sich der Ausgang auf einer Höhe größer als jene des Eingangs befindet.

3. Anlage nach einem der vorstehenden Ansprüche, **dadurch gekennzeichnet, dass** sie weiter Flotationsmittel (52, 17; 45, 27) der Sedimente umfasst, die in der Nachbarschaft des Bodens mindestens eines Kamins vorgesehen sind.

4. Anlage nach dem vorstehenden Anspruch, **dadurch gekennzeichnet, dass** die Flotationsmittel der Sedimente mindestens eine Leitung umfassen, die dazu bestimmt ist, ein Mikrooxygenationsgas, vorteilshalber ein anaerobes Gas, wie ein Biogas, Stickstoff oder Kohlendioxid zuzuführen, wobei diese Leitung mindestens eine Mikrooxygenationsvorrichtung versorgt, die in der Nachbarschaft des Bodens eines jeweiligen Kamins vorgesehen ist.

5. Anlage nach einem der vorstehenden Ansprüche, **dadurch gekennzeichnet, dass** die Anlage in ihrer thermophilen oder mesophilen Konfiguration Mittel zum Erhitzen der Behälter umfasst.

6. Anlage nach dem vorstehenden Anspruch, **dadurch gekennzeichnet, dass** die Mittel zum Erhitzen mindestens ein Netz an strahlenden Kanalisationen umfassen, die an den Innenwänden der Ringe und/oder am Boden der Ringe und/oder am Boden der Kamine angeordnet sind.

7. Anlage nach einem der vorstehenden Ansprüche, **dadurch gekennzeichnet, dass** sie einen einzigen ersten Behälter (1) umfasst, der ein einziges zentrales Fach (11) und ein einziges peripheres Fach (12), sowie einen einzigen zweiten Behälter (2), der der ein einziges zentrales Fach (21) und ein einziges peripheres Fach (22) beinhaltet.

8. Verfahren zur Anwendung einer Anlage zur Behandlung von biologischen Abfällen nach einem der vorstehenden Ansprüche, wobei das Verfahren die folgenden Schritte umfasst:
- Aufnehmen von zu behandelnden Abfällen im ersten Behälter (1),
- Durchführen mindestens eines Teils eines Hydrolyseschrittes im ersten Ring (12), und mindestens eines Teils eines Versäuerungsschrittes in dem ersten Kamin (11),
- Transfer der teilweise behandelten Abfälle aus dem ersten Kamin in den zweiten Ring (22),
- Durchführen mindestens eines Teils eines Versäuerungsschrittes in dem zweiten Ring (22), und mindestens eines Teils eines Methangasbildungsschrittes in dem zweiten Kamin (21),
- Austragen der behandelten Abfälle aus dem zweiten Kamin,
- wobei bei dem Verfahren Zirkulationsmittel (43, 44) derart aktiviert werden, dass die zu behandelnden Abfälle nacheinander aus dem ersten Volumen, das durch den ersten Ring (12) gebildet wird, in ein zweites Volumen, das durch den ersten Kamin (11) gebildet wird, und danach in ein drittes Volumen, das durch den zweiten Ring (22) gebildet wird, danach in ein viertes Volumen, das durch den zweiten Kamin (21) gebildet wird, transferiert werden, bevor sie aus dem zweiten Kamin (21) durch eine Kanalisation ausgehend von dem zweiten Behälter (2) dank einer halbkontinuierlichen Zuführung mit pneumatischer Forcierung ausgetragen werden,
- und wobei bei dem Verfahren eine Fraktion von in dem zweiten Behälter vorhandenen Abfällen ausgetragen werden, danach eine andere Fraktion an Abfällen in dem ersten Behälter aufgenommen wird, wobei die ausgetragene Fraktion und die aufgenommene Fraktion im Wesentlichen dieselben Volumina aufweisen.

9. Verfahren nach Anspruch 8, **dadurch gekennzeichnet, dass** für jeden Behälter (1, 2) unterschiedliche Aufnahmekapazitäten, und somit dem zentralen Fach (11, 21) und dem peripheren Fach (12, 22) unterschiedliche biologische Rückhaltedauern zugewiesen werden.

## Claims

1. Facility for treating organic waste, in order to produce biogas and recover at least one portion of the biogas produced, said facility comprising:
- a first tank (1) comprising first separation means (15) extending over a portion of the height of the tank, so as to define a first central compartment, or tube (11), a first peripheral compartment, or ring (12), closed in its upper portion, as well as a first compartment for stirring and biochemical exchanges (16) exclusively providing the communication between these two compartments, in the bottom portion of the tank, the first compartment for stirring comprising first stirring means (17),
- a second tank (2) comprising second separation means (25) extending over a portion of the height of the tank, so as to define a second central compartment, or tube (21), a second peripheral compartment, or ring (22), closed in its upper portion, as well as a second compartment for stirring and biochemical exchanges (26) exclusively providing the communication between these two compartments, in the bottom portion of the tank, the second compartment for stirring comprising second stirring means (27),
- means (ALIM) for feeding the waste to be treated, into the first ring,
- means (T) for transferring the partially treated waste, from the first tube (11) to the second ring, in such a way that the first tube (11) communicates at the upper portion exclusively with the top of the second ring (22), by a pipe,
- means (EVAC) for discharging the treated waste, out of the second tube,
- means (43, 44) for circulating the waste by pneumatic forcing, from the first ring (12) to the second tube (21), these means for circulating comprising:
- - a circulation gas line (43) opening into the upper portion of the first ring (12) and making it possible to create a sufficient pressure in the gas blanket of the ring (12) in order to cause to rise in the first tube (11) by pneumatic forcing a volume of substrate corresponding to that of the incoming materials, and
- - a circulation gas line (44) opening into the upper portion of the second ring (22) likewise making it possible to proceed by pneumatic forcing in discharging from the second tube (21) a volume of digestates corresponding to that of the incoming materials;
where the facility is therefore arranged in such a way that the waste to be treated is transferred in succession from the first volume formed by the first ring (12), to a second volume formed by the first tube (11), then to a third volume formed by the second ring (22), then to a fourth volume formed by the second tube (21), before being discharged from the second tube (21) by a pipe from the second tank (2), thanks to a semi-continuous feed with pneumatic forcing.

2. Facility according to claim 1, **characterised in that** the means for transferring the partially treated waste comprise said pipe, which forms a transfer line connecting an outlet of the first tank and an inlet of the second tank, said outlet being at an altitude that is higher than that of said inlet.

3. Facility as claimed in any preceding claim, **characterised in that** it further comprises means for floating (52, 17; 45, 27) sediments, provided in the vicinity of the bottom of at least one tube.

4. Facility as claimed in the preceding claim, **characterised in that** the means for floating sediments comprise at least one duct intended to convey a micro-bubbling gas, advantageously an anaerobic gas such as a biogas, nitrogen or carbon dioxide, with this duct supplying at least one micro-bubbling device provided in the vicinity of the bottom of a respective tube.

5. Facility as claimed in any preceding claim, **characterised in that**, in its thermophilic or mesophilic configuration, the facility comprises means for heating tanks.

6. Facility as claimed in the preceding claim, **characterised in that** the means for heating comprise at least one network of radiant pipes arranged on the inner walls of the rings and/or on the bottom of these rings and/or on the bottom of the tubes.

7. Facility as claimed in any preceding claim, **characterised in that** it comprises a single first tank (1) comprising a single central compartment (11) and a single peripheral compartment (12), as well as a single second tank (2) comprising a single central compartment (21) and a single peripheral compartment (22).

8. Method for implementing a facility for treating organic waste according to one of the preceding claims, said method comprising the following steps:
- waste to be treated is admitted into the first tank (1),
- at least one portion of a step of hydrolysis is carried out in the first ring (12) and at least one portion of a step of acidogenesis in the first tube (11),
- the partially treated waste is transferred from the first tube to the second ring (22),
- at least one portion of a step of acetogenesis is carried out in the second ring (22) and at least one portion of a step of methanogenesis in the second tube (21),
- the treated waste is discharged, out of the second tube.
- method wherein the means for circulating (43, 44) are activated, in such a way that the waste to be treated is transferred in succession from the first volume formed by the first ring (12), to a second volume formed by the first tube (11), then to a third volume formed by the second ring (22), then to a fourth volume formed by the second tube (21), before being discharged from the second tube (21) by a pipe from the second tank (2), thanks to a semi-continuous feed with pneumatic forcing,
- and method wherein furthermore a fraction of the waste present in the second tank is discharged, then another fraction of waste is admitted into the first tank, with the discharged fraction and the admitted fraction having substantially the same volumes.

9. Method according to claim 8, **characterised in that**, for each tank (1, 2), different receiving capacities and therefore different biological retention times are assigned to the central compartment (11, 21) and to peripheral compartment (12, 22).
